# EUROPÄISCHE PATENTSCHRIFT

(11) **EP 1 042 689 B1**
(45) Veröffentlichungstag und Bekanntmachung des Hinweises auf die Patenterteilung: **03.03.2004**
(21) Anmeldenummer: 98966282.0
(22) Anmeldetag: 14.12.1998
(51) Int. Cl.: G01T 1/17, G01T 1/29

(54) **ANORDNUNG ZUR DIGITALEN SUBTRAKTIONS-ANGIOGRAPHIE**
DEVICE FOR DIGITAL SUBTRACTION ANGIOGRAPHY
DISPOSITIF POUR REALISER UNE ANGIOGRAPHIE NUMERIQUE

(30) Priorität: 22.12.1997 DE 19758363
(43) Veröffentlichungstag der Anmeldung: 11.10.2000
(73) Patentinhaber: Deutsches Elektronen-Synchrotron DESY, 22603 Hamburg (DE)
(72) Erfinder: BESCH, Hans, Jürgen, D-57250 Netphen (DE); LOHMANN, Michael, D-22115 Hamburg (DE)
(74) Vertreter: UEXKÜLL & STOLBERG
(86) Internationale Anmeldenummer: PCT/EP1998/008037
(87) Internationale Veröffentlichungsnummer: WO 1999/032901

(56) Entgegenhaltungen:
- EP-A- 0 306 798
- DE-A- 3 901 837
- DE-C- 3 517 101

## Beschreibung

Die Erfindung betrifft eine Anordnung gemäß Oberbegriff des Patentanspruchs 1.

Eine derartige Anordnung ist bereits aus der DE 35 17 101 C1 bekannt. Sie dient zur Untersuchung des Herzens mit dem Ziel der Feststellung, ob ein akuter Verschluß eines Herzkranzgefäßes durch ein Blutgerinsel zu befürchten ist. Zu diesem Zweck wird dem Patienten ein Jodkontrastmittel in eine Armvene injiziert und der Patient wird zeilenweise gleichzeitig mit zwei zeilenförmig kollimierten Röntgenstrahlen bestrahlt, von denen der eine eine Energie E₁ knapp unterhalb der Jod-Absorptionskante von 33 keV und der andere eine Energie E₂ knapp oberhalb der Jod-Absorptionskante hat. Die beiden Röntgenstrahlen werden auf das Herz eines Patienten fokussiert und treffen dahinter auf einen Detektor mit zwei parallel im Abstand zueinander angeordneten Zählkammern, deren Signale über einen Ladungsempfindlichen A/D-Wandler in digitale Signale umgesetzt und einem Rechner zugeleitet, der dann jeweils ein Bild der Energie E₁ und ein Bild der Energie E₂ zusammensetzt und die Bilder logarithmisch voneinander subtrahiert. Das erhaltene Bild zeigt er auf einem Monitor an.

Die DE 39 01 837 A1 offenbart einen Strahlungsdetektor, der in der Lage ist, die örtliche Verteilung hoher Strahlintensitäten mit hoher Genauigkeit, einem großen dynamischen Bereich und hoher Empfindlichkeit in kurzen Aufnahmezeiten zu vermessen. Anwendungen liegen zum Beispiel in der medizinischen Diagnostik für Momentaufnahmen schnellbewegter Teile (Herzkranzgefäße). Hierzu werden gepulste Strahlungsquellen verwendet, wobei die Signale der Einzelquanten, die zu einem Strahlimpuls gehören, in einer Proportionalkammer summiert werden und diese so gewonnenen Einzelsignale pro Strahlungsquellen-Puls entweder bereits das gesuchte Intensitätssignal darstellen oder elektronisch pro Bildpunkt über eine Anzahl von Strahlungsquellen-Pulsen aufsummiert werden. Die DE 39 01 837 A1 zeigt weiterhin den konstruktiven Aufbau eines solchen Detektors. Der Nachteil dieses Detektors besteht jedoch darin, daß sich zusammen mit herkömmlichen Verstärker- und Wandlerschaltungen die erforderliche Bildauflösung nicht erreichen läßt.

Aufgabe der Erfindung ist es, die Anordnung der eingangs genannten Art dahingehend zu verbessern, daß die Auflösung des erhaltenen Bildes besser ist, damit insbesondere Herzkranzgefäße deutlicher dargestellt werden können.

Zur Lösung dieser Aufgabe dient eine Anordnung mit den Merkmalen des Patentanspruchs 1.

Sie enthält einen hochempfindlichen Verstärker und Analog-Digital-Wandler mit sehr hoher Dynamik, mit dem die hohen Absorptionsunterschiede im jodgefüllten Körper linear dargestellt werden können. Auf diese Weise ist es möglich, alle drei Herzkranzgefäße trotz Überlagerung durch jodgefüllte Herzkammern darzustellen.

In diesem Zusammenhang wird darauf hingewiesen, daß die Abbildung der Herzkranzgefäße deswegen so schwierig ist, weil sich einerseits das Herz andauernd bewegt und andererseits das Kontrastmittel sowohl in die Herzkammern als auch in die Herzkranzgefäße gelangt.

Zur Lösung der gestellten Aufgabe war es also erforderlich, für die verwendeten elektronischen Schaltungen einen geeigneten Baustein in Form eines Strom- bzw. Ladungs-Digital-Wandlers zu finden, der einen dynamischen Bereich von mindestens 18 Bit hat, um eine hinreichend gute Auflösung in Form von Farb- oder Kontrastunterschieden zu erreichen.

Die Erfindung wird im folgenden anhand von Figuren näher erläutert; es zeigt
- Figur 1: eine schematische Anordnung der Vorrichtung im Strahlengang der Röntgenstrahlung eines Synchrotrons;
- Figur 2: eine schematische Schnittansicht des bei der Anordnung gemäß Figur 1 verwendeten Detektors; und
- Figur 3: ein Schaltbild einer in dem Detektor aus den Figuren 1 und 2 verwendeten elektronischen Schaltung.

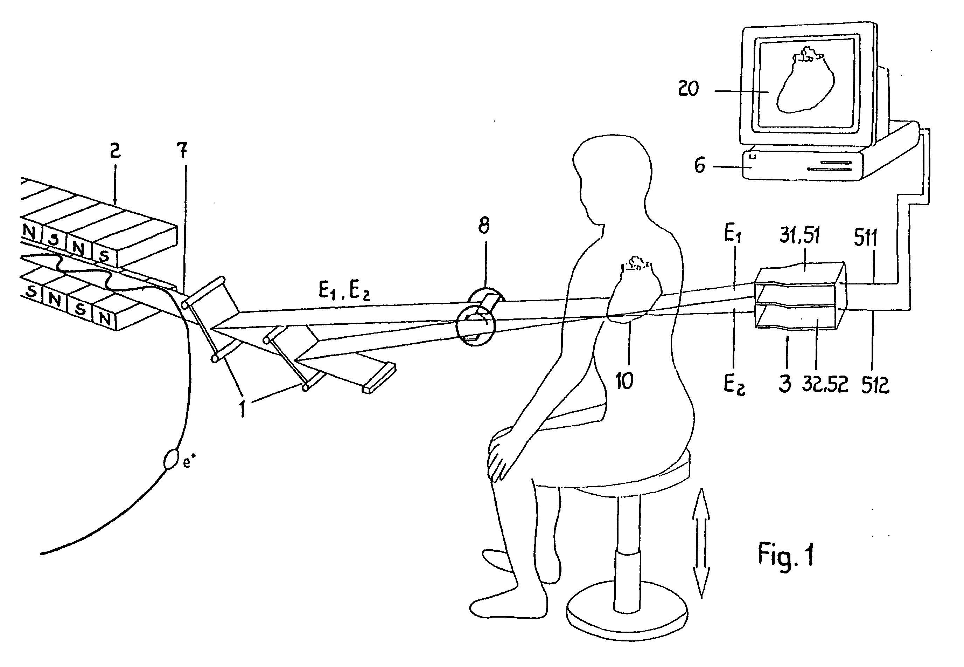

Figur 1 zeigt als Röntgenstrahlungsquelle beispielsweise einen Speicherring, wie er als DORIS bei der Anmelderin vorhanden ist. Dabei wird der umlaufende Positronenstrahl e⁺ zwischen den Polen eines sogenannten Wiggler-Magneten etwa in der dargestellten Weise durch jeweils hintereinander geschaltete, in der Polarität aber umgekehrte Polpaare in einer Ebene hin und her abgelenkt, was zu einer starken Synchrotronstrahlungsbildung führt. Diese Synchrotronstrahlung ist ein polychromatischer oder "weißer" Strahl 7, der durch ein nicht dargestelltes System von Kollimatoren und Blenden auf einen Monochromator 1 geleitet wird. Der Monochromator 1 befindet sich im Falle von DORIS etwa 15 bis 36m von dem Wiggler-Magneten 2 entfernt, der den Quellpunkt der Strahlung bildet. Der "weiße" Strahl 7 hat in der Nähe seines Quellpunktes einen etwa elliptischen Querschnitt, dessen kleine Achse etwa 2 mm lang ist, während die Länge der horizontal liegenden großen Achse etwa 4 mm beträgt. Durch Strahlblenden und natürliche Divergenz hat der Strahl 7 am Ort des Monochromators 1 eine horizontale Breite von ungefähr 100mm und eine Höhe von ungefähr 2,5mm. Durch Verwendung eines zweifachen Monochromators 1 entstehen daraus zwei monochromatische Strahlen mit einer Energie von E₁ und E₂. Die monochromatischen Strahlen E₁ und E₂ gelangen an den Eingang eines Detektors 3, wobei sie auf ihrem Wege im Betrieb das Herz 10 eines Patienten durchlaufen. Am Eingang des Detektors 3 haben sie einen Abstand von 1,5mm und eine horizontale Breite von zur Zeit 120mm und jeweils eine Höhe von 1,0mm.

Der Detektor 3 weist zwei Ionisationskammern 31, 32 mit angeschlossenen Nachweis-Schaltungen 51, 52 auf. Die Ausgangssignale aus den Nachweis-Schaltungen 51, 52 des Detektors 3 werden über Leitungen 511, 512 an ein Rechnersystem 6 übertragen, das die Bildauswertung in an sich bekannter Weise, beispielsweise gemäß DE 35 17 101 C1 steuert, indem es jeweils ein Bild der Energie E₁ von einem zweiten Bild der Energie E₂ abzieht und das erhaltene Bild auf einem Monitor 20 anzeigt.

Im Betrieb der erfindungsgemäßen Anordnung sitzt ein Patient auf einem Stuhl 9, der sich durch eine Hydraulik in gesteuerter Weise aufwärts und abwärts bewegen läßt. Dies ist durch den Doppelpfeil angedeutet. In einer Ausführungsform führt der Stuhl 9 eine Aufwärtsbewegung von etwa 40 cm durch, wobei die ersten 10 cm zur Beschleunigung des Stuhls 9 und des darauf sitzenden Patienten dienen, die anschließenden 20 cm seines Weges zur Bewegung mit konstanter Geschwindigkeit von 50 cm/sec und die letzten 10 cm zur Abbremsung dienen. Dadurch wird das zu untersuchende Organ des Patienten, beispielsweise das Herz 10, in einer Zeit von 250 msec durch die beiden monochromatischen Strahlen E₁ und E₂ bewegt. Ein und derselbe Untersuchungsort wird dabei so schnell hintereinander mit dem Strahl E₁ und dem Strahl E₂ abgebildet, daß sich die beiden Strahlbilder im Rechnersystem 6 ohne weiteres subtrahieren lassen.

Gemäß Figur 1 ist im Strahlengang der beiden Röntgenstrahlen zwischen dem Monochromator 1 und dem Detektor 3 noch vor dem Schnittpunkt der beiden Strahlen E₁, E₂ und damit vor dem zu untersuchenden Herzen 10 ein Sicherheitssystem 8 vorgesehen, das sehr schnelle Strahlverschlüsse aufweist, die die Röntgenstrahlen E₁ und E₂ in weniger als 10 msec absperren können. Derartige Sicherheissysteme werden beim Betrieb unseres Synchrotrons seit vielen Jahren eingesetzt.

Die Steuerung des Stuhls 9 erfolgt ebenfalls über das Rechnersystem 6, ohne daß dies in der Zeichnung besonders angedeutet ist. Es bereitet dem Fachmann jedoch keine Schwierigkeiten, die nicht dargestellte Hydraulik für das Anheben und Absenken des Stuhls 9 von dem Rechnersystem 6 zu steuern.

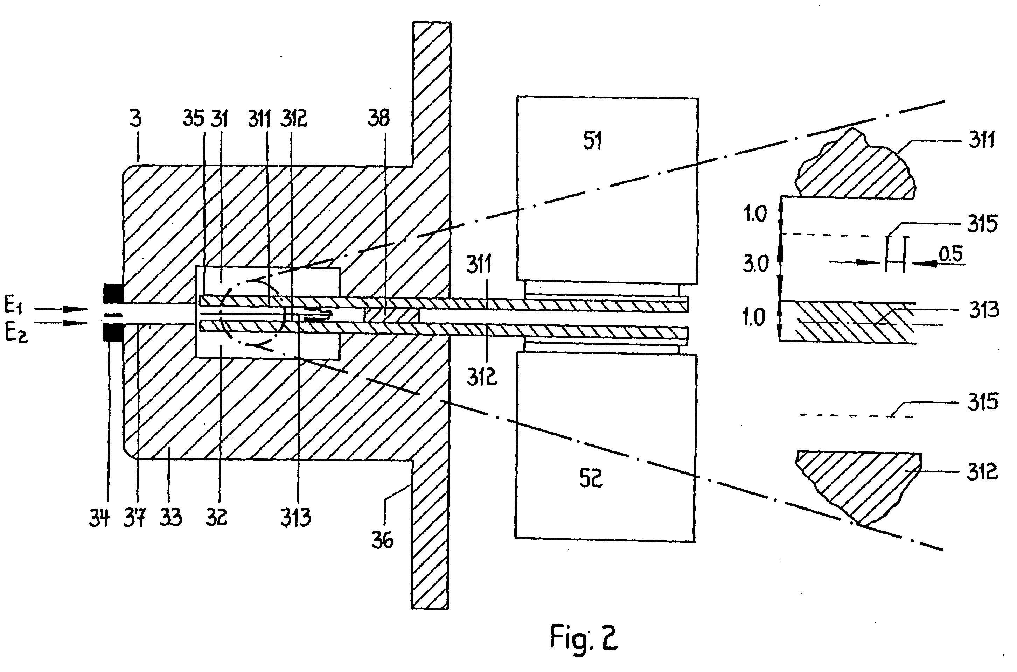

Figur 2 zeigt einen Vertikalschnitt durch den Detektor 3 von Figur 1, der aus zwei Ionisationskammern 31, 32 mit je einer Platine 311 und 312 und einer gemeinsamen Driftkathode 313 gebildet ist.

Die beiden Ionisationskammern 31, 32 sind von einem Gehäuse 33 umschlossen, das im wesentlichen rechteckförmig ist und an einer Seite einen Befestigungsflansch 36 aufweist. Ein Einlaßkanal 37 von etwa 10mm Höhe, 150mm Breite und etwa 30mm Länge durchsetzt eine Wand des Gehäuses 33 und trägt an seinem freien Ende einen an sich bekannten Kollimator 34, durch den die beiden Strahlen E₁ und E₂ eintreten können. Das innere Ende des Kanals 37 ist durch ein Kohlefaserfenster (35) verschlossen.

Im Inneren des Gehäuses 33 befindet sich ein Hohlraum, der mit einem Ionisationsgas wie Krypton oder Xenon und einem Löschgas, z.B. Kohlendioxid unter einem Druck von 10 bis 20 bar gefüllt ist. Das Partialdruckverhältnis von Ionisationsgas zu Löschgas beträgt etwa 90:10.

Wie bereits erwähnt weist jede Ionisationskammer 32 eine glasfaserverstärkte Platine 311, 312 auf, die etwa im Abstand von 9mm zueinander angeordnet sind. An den einander zugewandten Seiten der Platinen 311, 312 sind vergoldete Kupferstreifen als Anodenstreifen angeordnet, die in Strahlrichtung verlaufen und im Raster von 400 µm angeordnet sind. Im rechten Teil von Figur 2 ist der durch einen strichpunktierten Kreis hervorgehobene Teil der Platinen 311, 312 vergrößert dargestellt. Man erkennt daraus, daß zwischen den die Anodenstreifen tragenden Platinen 311, 312 eine gemeinsame Driftkathode 313 angeordnet ist und daß in dem Raum von der Driftkathode 313 zu der ersten bzw. zweiten Platine 311, 312 ein sogenanntes "Frisch-Gitter" angeordnet ist, das näher zu den Platinen 311, 312 liegt. In einer Ausführungsform hat die Driftkathode 313 eine Dicke von 1,0mm, während der Abstand zwischen der Driftkathode 313 und jeder Platine 311, 312 4,0 mm beträgt. Die beiden Frisch-Gitter 315 sind dann jeweils im Abstand von 1,0mm zu den Platinen 311, 312 und damit im Abstand von jeweils 3,0mm zur Oberfläche der Driftkathode 313 angeordnet. Frisch-Gitter bestehen aus speziellen Drähten im Abstand von 0,5mm die die in den Ionisationskammern 31, 32 gebildeten Ionen gegenüber den Anodenstreifen abschirmen.

Die ersten und zweiten Platinen 311, 312 werden in Strahlrichtung aus dem Gehäuse 33 des Detektors 3 herausgeführt und sind an ihren Enden jeweils mit einer Nachweis-Schaltung 51, 52 verbunden. Es ist klar, daß das Gehäuse 33 des Detektors 3 verschlossen sein muß, um das Ionisationsgas und das Löschgas einschließen zu können. Zu diesem Zweck ist zwischen die Platinen 311, 312 ein Stopfen 38 eingebracht, der die beiden Platinen 311, 312 gasdicht miteinander verbindet, beispielsweise dadurch, daß er mit den Platinen 311, 312 verklebt ist. Die beiden Platinen 311, 312 sind ihrerseits mit der Wand des Gehäuses 33 gasdicht verbunden, beispielsweise ebenfalls durch Verkleben.

Wie bereits erwähnt, werden die auf den einander zugewandten Seiten der Platinen 311, 312 angeordneten Anoden als vergoldete Kupferstreifen im Raster von 400 µm aus dem Gehäuse 33 des Detektors 3 herausgeführt, und zwar in Form von 336 parallelen Streifen, die im Abstand von etwa 0,3mm angeordnet sind und die jeweils eine Breite von etwa 0,4mm haben. Die Länge jeder Platine 311, 312 beträgt etwa 230mm, während die Länge der Ionisationskammern 31, 32 etwa 60mm ausmacht.

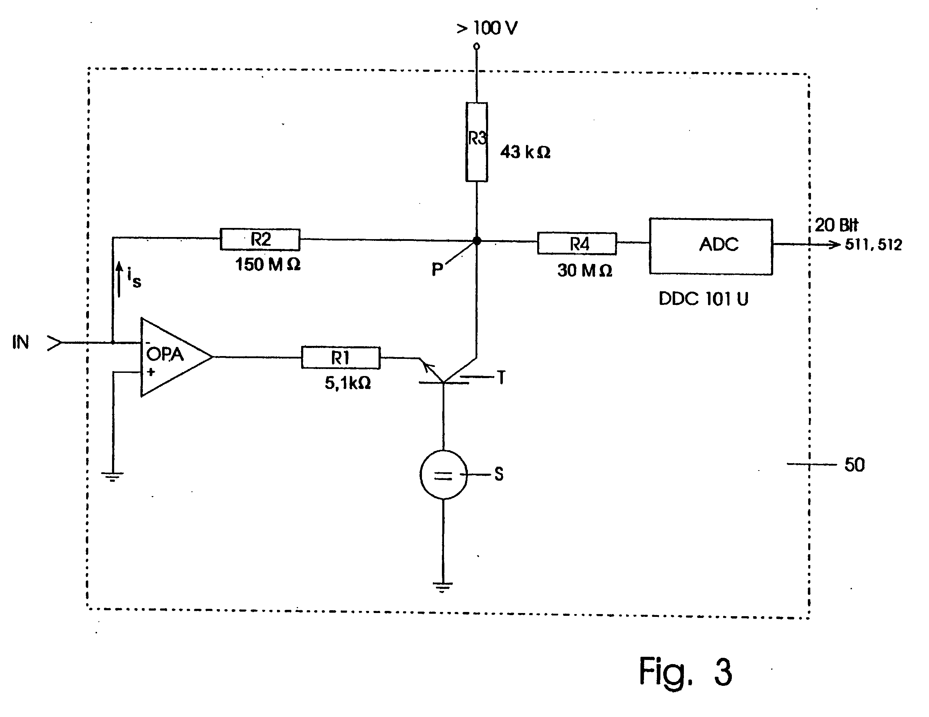

Im Inneren der Ionisationskammern 31, 32 verlaufen die Anodenstreifen über eine Länge von etwa 56mm parallel, während sie außerhalb der Ionisationskammern 31, 32 divergieren und insbesondere außerhalb des Gehäuses 33 des Detektors 3 zu einem derartigen Abstand aufgeweitet sind, daß in der Nachweis-Schaltung 51 bzw. 52 an jeden Anodenstreifen elektronische Schaltung 50. gemäß Figur 3 angeschlossen werden kann. Die beiden Nachweis-Schaltungen 51 und 52 enthalten also in einer Ausführung 2 x 336 = 672 elektronische Schaltungen 50, die den in Figur 3 gezeigten Aufbau haben.

Figur 3 zeigt eine der 2 x 336 elektronische Schaltungen 50, deren Eingang IN an einen der 2 x 336 Anodenstreifen der Platinen 311 oder 312 angeschlossen ist. Der Eingang IN der elektronische Schaltung 50 führt an den negativen Eingang eines Operationsverstärkers OPA, dessen positiver Eingang auf Masse liegt. Der Operationsverstärker OPA ist ein Typ OPA 129 UB, der von der Firma Burr-Brown hergestellt wird. Er wird normal mit ± 15 V betrieben und ist ein sehr rauscharmer Operationsverstärker.

An den Ausgang des Operationsverstärkers OPA ist ein Transistor T mit seinem Emmitter angeschlossen, und zwar über einen ersten Widerstand R₁ von 5,1 kΩ. An der Basis des Transistors T liegt eine Gleichspannungsquelle S, die eine Gleichspannung von -4 V zur Verfügung stellt. Der Kollektor des Transistors T liegt an einem Punkt P, der im Betrieb Spannungen von 0 bis etwa 90 V sieht. Aus diesem Grunde ist der Transistor T ein spannungsfester Transistor, der als Basisschaltung eingesetzt wird. Der Kollektor des Transistors T koppelt über den Punkt P und einen zweiten Widerstand R₂ von 150 MΩ auf den negativen Eingang des Operationsverstärkers OPA zurück.

An den Punkt P der elektronische Schaltung 50 wird eine hohe Gleichspannung von 100 V oder mehr gelegt, und zwar über einen dritten Widerstand R₃ von beispielsweise 43 kΩ.

Der Operationsverstärker OPA hält die Differenzspannung an seinem Eingang auf nahezu Null Volt, so daß der Strom iₛ vom Eingangssignal über den Widerstand R₂ zum Punkt P und von dort über einen vierten Widerstand R₄ von 30 MΩ an einem Analog-Digital-Wandler ADC fließt. Der Analog-Digital-Wandler ist ein Baustein des Typs DDC 101 U der Firma Burr-Brown mit einer Auflösung von 20 Bit. Dieser Chip wurde speziell zum Auslesen von Photodioden konzipiert, bei denen die positiven Ladungen oder Löcher ausgelesen werden. In seinem unipolaren Betrieb ist er daher nur für die Umwandlung positiver Ladungssignale in 20 Bit-Signale einsetzbar. Der Chip DDC 101 U kann zwar auch im bipolaren Betrieb eingesetzt werden, dies reduziert die Ausgabe jedoch auf 19 Bit. In diesem Betrieb ist das Rauschen des Chips so hoch, daß man ihn für den vorliegenden Fall der Bilddarstellung nicht verwenden kann.

Aus diesem Grunde kommt für die vorliegende Anwendung nur der unipolare Betrieb des Chips DDC 101 U in Frage, allerdings besteht dann die Schwierigkeit darin, daß der Chip nur positive Ladungen (Löcher) verarbeiten kann, während von den Ionisationskammern 31, 32 über die Anodenstreifen nur negative Ladungen, nämlich Elektronen, geliefert werden. Für die Erfindung ist daher wesentlich, daß aus den negativen Ladungen, die am Eingang des Operationsverstärkers OPA ankommen, positive Ladungen gemacht werden, die von dem speziellen Analog-Digital-Wandler ADC des Typs DDC 101 U verarbeitet werden können. Dazu ist es zunächst erforderlich, den am Eingang des Analog-Digital-Wandlers liegenden Widerstand R₄ > 20 MΩ zu machen, denn bei kleineren Widerständen als 20 MΩ erzeugt der Chip DDC 101 U ein größeres Rauschen als das Widerstandsrauschen, das auch als Nyquist-Rauschen bezeichnet wird. Wenn das Rauschen aber bereits 4 Bit produziert, dann bleiben nur noch 16 Bit zur Bilddarstellung übrig, was zu wenig ist, um die erforderliche Bildauflösung zu erzielen.

Wählt man für den Widerstand R₄ aber mehr als 20 MΩ, dann wird eine entsprechend höhere Ansteuerungsspannung am Punkt P benötigt, um den DDC 101 U in etwa 0,2 msec auf 20 Bit aufzuladen. Wählt man für den Widerstand R₄ beispielsweise 30 MΩ, dann müßte die Aussteuerungsspannung am Punkt P auf bis zu 90 Volt oder mehr ansteigen können. Eine so hohe Ansteuerungsspannung kann zur Zerstörung des Operationsverstärkers OPA führen, da er nur maximal 20 bis 36 Volt vertragen kann.

Aus diesem Grunde wird der spannungsfeste Transistor T in Basisschaltung zwischen den Operationsverstärker OPA und den Punkt P gelegt. Die Basisschaltung bewirkt bekanntlich eine Spannungsverstärkung, wobei der Transistor T genau so stark aufsteuert, daß der Spannungsabfall über den zweiten Widerstand R₂ dafür sorgt, daß die Spannungsdifferenz am Eingang des Operationsverstärkers OPA Null bzw. nahezu Null ist. Dies hat zur Folge, daß die am Operationsverstärker OPA eintreffenden Ladungen auch am Eingang des Analog-Digital-Wandlers eintreffen, und zwar in fünffacher Verstärkung, da der Widerstand R₂ fünfmal so groß wie der Widerstand R₄ gewählt ist. In einer bevorzugten Ausführungsform ist nämlich R₂ = 150 MΩ, R₄ = 30 MΩ.

Das Wesen der erfindungsgemäßen elektronischen Schaltung 50 besteht also in folgenden Maßnahmen:
1. Negative Ladungen werden in positive Ladungen umgewandelt, um von dem Chip DDC 101 U verarbeitet werden zu können.
2. Die Ansteuerungsspannung für den ADC-Chip wird auf bis zu 90 Volt oder mehr gewählt.
3. Die hohe Ansteuerungsspannung für den ADC wird durch eine Basisschaltung vom Operationsverstärker abgehalten.

Dadurch wird ein Signal: Rausch - Verhältnis von 300.000 : 1 erreicht, was für die Klarheit der Abbildung für den diagnostizierenden Arzt ausreicht. Die von den Ionisationskammern 31, 32 auf den Anodenstreifen gelieferten Signalströme liegen im übrigen im Bereich von 100 fA oder weniger, also iₘₐₓ < 100 x 10⁻¹⁵A. Diese Ströme sind so gering, daß sie einzelnen 33keV Photonen entsprechen.

## Patentansprüche

1. Anordnung zur digitalen Subtraktions-Angiographie im Energiesubtraktions-Modus,
- mit einem Monochromator (1) zur Erzeugung von zwei monochromatischen Röntgenstrahlen (E₁, E₂);
- mit einem Sicherheitssystem (8) mit sehr schnellen Strahlverschlüssen;
- mit einer Line Scan Einrichtung, angetrieben durch eine Hydraulik, auf der ein auf und ab bewegbaren Stuhl (9) zur Positionierung eines Patienten montiert ist;
- mit einem zweizeiligen Detektor (3);
- mit einem Rechnersystem (6) für Systemkontrolle, Datennahme und Bildverarbeitung;
**dadurch gekennzeichnet,**
- **daß** der Detektor (3) von zwei ortsauflösenden Ionisationskammern (31, 32) gebildet ist, die mit einem Ionisationsgas gefüllt sind und die eine bestimmte Anzahl von Anodenstreifen (311, 312) aufweisen;
- **daß** für beide Ionisationskammern (31, 32) eine gemeinsame Driftkathode (313) verwendet wird;
- **daß** an jede Ionisationskammer (31, 32) eine separate Nachweis-Schaltung (51, 52) mit je einer elektronischen Schaltung (50) für jeden Anodenstreifen (311, 312) angeschlossen ist, die als ein Signal-Wandler zwischen 0 Volt und 175 Volt linear arbeiten;
- **daß** jede elektronische Schaltung (50) an ihrem Eingang einen Operationsverstärker (OPA) aufweist, an dessen negativen Eingang das Eingangssignal von den Anodenstreifen (311, 312) gelegt wird, während der positive Eingang an Masse liegt;
- **daß** der Ausgang des Operationsverstärkers (OPA) über einen ersten Widerstand (R₁) mit dem Emitter eines Transistors (T) verbunden ist, an dessen Basis eine Gleichspannungsquelle (S) liegt und dessen Kollektor über einen Punkt (P) und einen vierten Widerstand (R₄) an den Eingang eines Analog/Digital-Wandlers (ADC) angeschlossen ist;
- **daß** der vierte Widerstand (R₄) größer als 20 MΩ ist;
- **daß** an den Punkt (P) über einen dritten Widerstand (R₃) eine Gleichspannung von 100 V oder mehr angelegt ist;
- **daß** zwischen dem Eingang des Operationsverstärkers (OPA) und dem Punkt (P) ein zweiter Widerstand (R₂) liegt, der den Eingangssignalstrom (iₛ) letztlich an den Analog/Digital-Wandler (ADC) leitet; und
- **daß** die Ausgaben aller elektronischen Schaltungen (50) als Bit-Worte über ein Bus-System (511, 512) an das Rechnersystem (6) übertragen werden.

2. Anordnung nach Anspruch 1, **dadurch gekennzeichnet, daß** der Analog/Digital-Wandler (ADC) ein 20 Bit Ladungs-Digital-Wandler ist.

3. Anordnung nach Anspruch 1 oder 2, **dadurch gekennzeichnet, daß** der vierte Widerstand (R₄) 30 MOhm und der zweite Widerstand (R₂) 150 MOhm hat.

4. Anordnung nach Anspruch 1, **dadurch gekennzeichnet, daß** der erste Widerstand (R₁) 5,1 kOhm und der dritte Widerstand (R₃) 43 kOhm hat.

5. Anordnung nach Anspruch 4, **dadurch gekennzeichnet, daß** an der vom Punkt (P) abgewandten Seite des dritten Widerstands (R₃) etwa +145V Gleichspannung liegen.

6. Anordnung nach einem der Ansprüche 1-5, **dadurch gekennzeichnet, daß** der als Basisschaltung betriebene Transistor (T) ein bis etwa 200V spannungsfester Transistor ist.

7. Anordnung nach Anspruch 6, **dadurch gekennzeichnet, daß** die Gleichspannungsquelle (S) eine Gleichspannung von weniger als -1V an die Basis des Transistors (T) legt.

8. Anordnung nach einem der Ansprüche 1-7, **dadurch gekennzeichnet, daß** der Operationsverstärker (OPA) von einer Difet-Schaltung gebildet ist.

9. Anordnung nach Anspruch 1, **dadurch gekennzeichnet, daß** der Detektor (3) für jede Energie (E₁, E₂) eine Anzahl von 336 Signalleitungen aufweist.

## Claims

1. Arrangement for digital subtraction angiography in the energy subtraction mode comprising a monochromator (1) for generating two monochromatic X-ray beams (E1, E2); comprising a safety system (8) with very rapid beam shutters; comprising a line scan device driven by a hydraulics system, on which a chair (9), which can be moved up and down for positioning a patient, is mounted; comprising a two-line detector (3); comprising a computer system (6) for system control, data acquisition and image processing; **characterised in that** the detector (3) is formed by two locally resolving ionisation chambers (31, 32), which are filled with an ionisation gas and which have a certain number of anode strips (311, 312); **in that** a common drift cathode (313) is used for both ionisation chambers (31, 32); **in that** a separate detection circuit (51, 52), each with an electronic circuit (50) for each anode strip (311, 312), is connected to each ionisation chamber (31, 32), which detection circuits operate linearly as a signal converter between 0 and 175 volts; **in that** each electronic circuit (50) has an operational amplifier (OPA) at its input, to the negative input of which the input signal from the anode strips (311, 312) is applied, while the positive input is earthed; **in that** the output of the operational amplifier (OPA) is connected via a first resistance (R₁) to the emitter of a transistor (T), at the base of which there is a DC voltage source (S), and the collector of which is connected via a point (P) and a fourth resistance (R₄) to the input of an analogue-digital converter (ADC); **in that** the fourth resistance (R₄) is greater than 20 MΩ; **in that** a DC voltage of 100 v or more is applied to the point (P) via a third resistance (R₃); **in that** between the input of the operational amplifier (OPA) and the point (P), there is a second resistance (R₂), which passes the input signal current (iₛ) finally to the analogue-digital converter (ADC); and **in that** the outputs of all the electronic circuits (50) are transmitted as bit words via a bus system (511, 512) to the computer system (6).

2. Arrangement according to claim 1, **characterised in that** the analogue-digital converter (ADC) is a 20-bit charge digital converter.

3. Arrangement according to either of claims 1 and 2, **characterised in that** the fourth resistance (R₄) has 30 MOhm and the second resistance (R₂) has 150 MOhm.

4. Arrangement according to claim 1, **characterised in that** the first resistance (R₁) has 5.1 kOhm and the third resistance (R₃) has 43 kOhm.

5. Arrangement according to claim 4, **characterised in that** there is approximately + 145 v DC voltage at the side of the third resistance (R₃) remote from point (P).

6. Arrangement according to any one of claims 1 to 5, **characterised in that** the transistor (T) operated as a base circuit is a transistor that is voltage-stable up to about 200 v.

7. Arrangement according to claim 6, **characterised in that** the DC voltage source (S) applies a DC voltage of less than -1 v to the base of the transistor (T).

8. Arrangement according to any one of claims 1 to 7, **characterised in that** the operational amplifier (OPA) is formed from a Difet circuit.

9. Arrangement according to claim 1, **characterised in that** the detector (3) has a number of 336 signal leads for each energy (E₁, E₂).

## Revendications

1. Installation pour l'angiographie soustractive numérique en mode de soustraction d'énergie, comportant
- un monochromateur (1) destiné à générer deux faisceaux de rayons X monochromatiques (E₁, E₂) ;
- un système de sécurité (8) comportant des obturateurs de faisceau très rapides ;
- un dispositif Line Scan, entraîné par un moyen hydraulique sur lequel est montée une chaise (8) mobile de haut en bas en vue de positionner un patient ;
- un détecteur biligne (3) ;
- un système informatique (6) pour le contrôle du système, l'acquisition de données et le traitement d'image ;
**caractérisée en ce que**
- le détecteur (3) est formé par deux chambres d'ionisation à résolution locale (31, 32) qui sont remplies de gaz d'ionisation et qui comportent un nombre déterminé de bandes anodiques (311, 312) ;
- une cathode de dérive commune (313) est utilisée pour les deux chambres d'ionisation (31, 32) ;
- un circuit de détection séparé (51, 52), doté d'un circuit électronique (50) pour chaque bande anodique (311, 312), est raccordé à chaque chambre d'ionisation (31, 32), lesdites bandes anodiques fonctionnant linéairement en convertisseur de signal entre 0 Volt et 175 Volts ;
- chaque circuit électronique (50) comporte à son entrée un amplificateur opérationnel (OPA) à l'entrée négative duquel est appliqué le signal d'entrée provenant des bandes anodiques (311, 312), tandis que l'entrée positive est mise à la masse ;
- la sortie de l'amplificateur opérationnel (OPA) est reliée via une première résistance (R₁) à l'émetteur d'un transistor (T) dont la base est reliée à une source de tension continue (S) et dont le collecteur est relié via un point (P) et une quatrième résistance (R₄) à l'entrée d'un convertisseur analogique/numérique (ADC) ;
- la quatrième résistance (R₄) est supérieure à 20 MΩ;
- une tension continue de 100 V ou plus est appliquée au point (P) via une troisième résistance (R₃) ;
- une deuxième résistance (R₂) est placée entre l'entrée de l'amplificateur opérationnel (OPA) et le point (P), ladite deuxième résistance amenant enfin le courant de signal d'entrée (iₛ) au convertisseur analogique/numérique (ADC) ; et
- les sorties de tous les circuits électroniques (50) sont transférées sous forme de mots binaires au système informatique (6) via un système de bus (511, 512).

2. Installation selon la revendication 1, **caractérisée en ce que** le convertisseur analogique/numérique (ADC) est un convertisseur numérique de charge de 20 bits.

3. Installation selon la revendication 1 ou 2, **caractérisée en ce que** la quatrième résistance (R₄) est de 30 MΩ et la deuxième résistance (R₂) est de 150 MΩ.

4. Installation selon la revendication 1, **caractérisée en ce que** la première résistance (R₁) est de 5,1 kΩ et la troisième résistance (R₃) est de 43 kΩ.

5. Installation selon la revendication 4, **caractérisée en ce que** la borne de la troisième résistance (R₃) qui est opposée au point (P) porte une tension d'environ + 145 V.

6. Installation selon la revendication 1 à 5, **caractérisée en ce que** le transistor (T) monté en base commune est un transistor à tension invariable d'environ 200 V.

7. Installation selon la revendication 6, **caractérisée en ce que** la source de tension continue (S) applique une tension continue inférieure à -1 V sur la base du transistor (T).

8. Installation selon l'une des revendications 1 à 7, **caractérisée en ce que** l'amplificateur opérationnel (OPA) est formé par un circuit Difet.

9. Installation selon la revendication 1, **caractérisée en ce que** le détecteur (3) comporte pour chaque énergie (E₁, E₂) 366 lignes de signal.
